# EUROPÄISCHE PATENTSCHRIFT

(11) **EP 0 927 011 B1**
(45) Veröffentlichungstag und Bekanntmachung des Hinweises auf die Patenterteilung: **22.01.2003**
(21) Anmeldenummer: 96927498.4
(22) Anmeldetag: 04.09.1996
(51) Int. Cl.: A61F 2/44, A61L 27/00

(54) **ZWISCHENWIRBEL-IMPLANTAT**
INTERVERTEBRAL IMPLANT
IMPLANT INTERVERTEBRAL

(43) Veröffentlichungstag der Anmeldung: 07.07.1999
(73) Patentinhaber: SYNTHES AG Chur, 7002 Chur (CH)
(72) Erfinder: WEBB, John, Nottingham (GB); KNOTHE, Inga, Maren, CH-2542 Pieterlen (CH); HAEFELI, Thomas, CH-2540 Grenchen (CH); BENOIT, Alfred, CH-2543 Lengnau (CH)
(74) Vertreter: Lusuardi, Werther Giovanni, Dr.
(86) Internationale Anmeldenummer: CH9600303
(87) Internationale Veröffentlichungsnummer: WO98009586

(56) Entgegenhaltungen:
- EP-A- 0 493 698
- EP-A- 0 505 634
- WO-A-88/03417
- DE-A- 4 423 826
- FR-A- 2 703 580
- US-A- 4 683 161
- US-A- 5 306 303
- US-A- 5 306 309

## Beschreibung

Die Erfindung betrifft ein Zwischenwirbel-Implantat gemäss dem Patentanspruch 1.

Solche Zwischenwirbel-Implantate werden bei der Fusion von Wirbelkörpern - nach der Entfernung der dazwischenliegenden Bandscheibe - eingesetzt, insbesondere im Bereich der lumbalen Wirbelsäule. Pro Zwischenwirbelraum werden ein bis zwei Implantate verwendet.

Aus der EP-B 346.269 FUHRMANN ET AL. ist bereits ein Zwischenwirbelimplantat bekannt, bei dem die nach aussen weisenden Stirn- und Seiten-Oberflächen des Implantats aus Hydroxyl-Apatit oder keramischem HIP-Material beschichtet sind. Nachteilig bei diesem bekannten Implantat ist der Umstand, dass der Grundkörper des Implantats aus üblichen nicht-keramischen und damit auch nicht-resorbierbaren Materialien besteht.

Aus der US-A-5 306 303 LYNCH ist bereits ein Zwischenwirbel-Implantat bekannt, welches vollständig aus einem porösen keramischen Material besteht. Nachteilig bei diesem bekannten Implantat ist jedoch einerseits die geringe Druckstabilität, die sich aus der relativ hohen Porosität ergibt und anderseits, dass sich das Implantat nicht mit Knochenspänen füllen lässt, um eine schnellere Knochen-Einbettung zu erzielen.

Aus der EP 505 634 OKA et al. ist ein weiteres Zwischenwirbelimplantat bekannt, welches aus einem porösen Keramik-Grundkörper mit in den Poren eingelagertem Hydrogel besteht. Auch bei diesem bekannten Implantat ist, wegen seiner mit Hydrogel gefüllten Poren, die Druckstabilität ungenügend.

Aus der EP-A-493 698 HÄRLE ist ein Knochenersatz zur Defektauffüllung bekannt, der aus zwei verschiedenen, porösen, keramischen Werkstoffen besteht, dessen Poren evakuiert sind.

Aus der DE-A 44 23 826 ASAHI eine keramische Wirbelprothese bekannt, deren Porosität durch ein Schäummittel erhalten wird. Schliesslich ist aus der US-A 5 306 309 WAGNER ET AL. ein gattungsgemässes Zwischenwirbelimplantat bekannt, welches jedoch nur eine grobe Strukturierung aufweist.

Hier will die Erfindung Abhilfe schaffen. Der Erfindung liegt die Aufgabe zugrunde, ein Zwischenwirbel-Implantat zu schaffen, welches eine verbesserte Lagestabilität aufweist und ein Anwachsen des Knochens in einer frühen Phase fördert.

Zur Lösung dieses Problems ist das eingangs genannte Implantat durch die Merkmale des kennzeichnenden Teils des unabhängigen Anspruchs 1 weitergebildet.

Damit ist der Vorteil erzielbar, dass das erfindungsgemässe Implantat - nach der erfolgten Primärfusion - die Distanz (entsprechend der Bandscheibenhöhe) zwischen den beiden Wirbelkörpern während des Resorptionsprozesses - bei gleichzeitig adäquater Fusion - ausgleicht und dass das Implantat nach einer bestimmten Zeit, wegen der auftretenden Resorption, im Körper nicht mehr nachweisbar ist.

Ein weiterer wesentlicher Vorteil ergibt sich auch aus der Röntgentransparenz des Implantats, welche störende Effekte bei der Beurteilung der umliegenden knöchernen Strukturen vermeidet.

Das Zwischenwirbelimplantat kann entweder als prismatischer oder zylindrischer Körper ausgebildet sein, mit einer Porosität von höchstens 30 Vol.-%. Gemäss einer bevorzugten Weiterbildung der Erfindung beträgt die Porosität des keramischen Werkstoffs höchstens 9 Vol.-%, vorzugsweise höchstens 5 Vol.-%. Dank der verringerten Porosität des Implantats ergibt sich eine hohe Druckfestigkeit, was vor allem im lumbalen Bereich der Wirbelsäule eine Grundvoraussetzung bildet. Eine möglichst grosse druckstabile Kontaktfläche von Endplatte zu Implantat ist hier wichtig. Deshalb sollte die Wandstärke des ringförmigen Zwischenwirbel-Implantats mindestens 4 mm, vorzugsweise mindestens 6 mm betragen, um einem Einsinken des Implantats in die Endplatten vorzubeugen.

Bei einer weiteren bevorzugten Ausführungsform der Erfindung beträgt die Dichte des keramischen Werstoffs mehr als 2,8, vorzugsweise mehr als 3,1, was die Druckfestigkeit des Implantats weiter verbessert.

Vorzugsweise ist das Implantat als hohler Kreiszylinder ausgebildet, um das Einbringen von körpereigenen Knochenspänen oder ähnlichen biokompatiblen Materialien zu ermöglichen und somit einer raschen Fusion des Implantats Vorschub zu leisten.

Gemäss einer weiteren bevorzugten Ausführungsform der Erfindung ist die Deckfläche und/oder die Grundfläche des Implantats nicht planar ausgebildet, sondern weist quer zur Zylinderachse des Implantats verlaufenden Rillen und/oder Erhöhungen auf. Diese dreidimensionale Strukturierung der Deck- und Grundfläche ermöglich direkt nach dem Einführung des Implantats in den Zwischenwirbelraum eine Primärverankerung, womit die Lagestabilität des Implantats , bzw. die Rotationsstabilität der benachbarten Wirbelkörper erhöht wird. Die dreidimensionale Strukturierung ist vorzugsweise in Form von "Wellen" (Erhöhungen, bzw. Versteifungen mit ausgeprägten Radien) in Längs- und Querrichtung ausgebildet.

Je nach Anwendungsbereich des Implantats ist die Deckfläche und/oder die Grundflächen parallel oder keilförmig zueinander zulaufend angeordnet, um in jedem Bereich der Wirbelsäule die Kurvenbildung adäquat nachformen zu können (Lordose, Kyphose).

Das Implantat besitzt vorzugsweise eine nach aussen gewölbte konvexe Deckfläche und/oder Grundfläche, welche der konkaven Formgebung der natürlichen Wirbelkörper-Endplatten angeglichen ist, um eine bessere Kontaktzone zwischen Implantat und Endplatten zu erreichen.

Vorzugsweise weist der Mantel des Zwischenwirbel-Implantats eine oder mehrere Perforationen auf, welche primär der Aufnahme eines Instrumentes zur Manipulation des Implantats dienen. Die Perforationen können sowohl an der anterioren Seite des Implantats, als auch in der lateralen Zone des Implantats angebracht werden. Im weiteren dienen die Perforationen in der Mantelfläche zur Förderung der primären knöchernen Durchbauung des Implantats.

Die Lagestabilität des Implantats kann noch dadurch verbessert werden, dass der Mantel des Zwischenwirbel-Implantat mit einer feinen dreidimensionalen Strukturierung versehen wird, welche das Anwachsen des Knochens in einer frühen Phase fördert. Diese Feinstrukturierung ist vorzugsweise 0,5 - 1,0 mm tief bei einer Rillenbreite von 0,5 - 1,0 mm. Die Anordnung der Strukturierung kann über die gesamte Mantelfläche erfolgen.

Für das erfindungsgemässe Implantat eignen sich die üblichen in der Medizin bereits erprobten keramischen Materialien mit der erfindungsgemäss definierten Porosität, wobei insbesondere polykristalline Keramiken bevorzugt werden, bei welchen der Fremdphasenanteil kleiner als 3 vorzugsweise kleiner als 2 Gew.-% ist. Die Druckfestigkeit des keramischen Werkstoffs beträgt zweckmässigerweise 400 - 600 MPa, vorzugsweise 450 - 550 MPa.

Die Erfindung und Weiterbildungen der Erfindung werden im folgenden anhand der teilweise schematischen Darstellungen eines Ausführungsbeispiels noch näher erläutert.

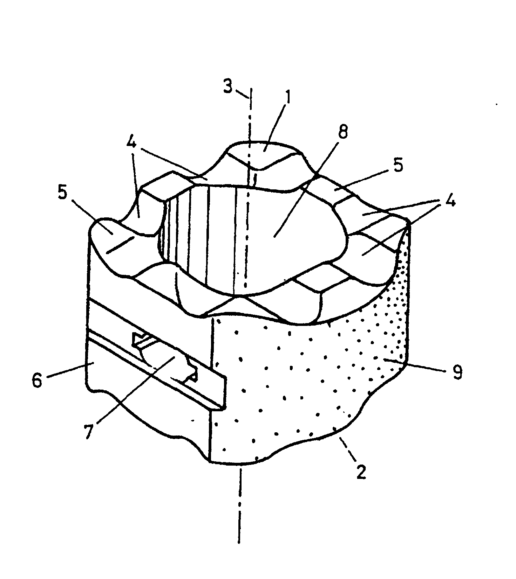

Die einzige Figur zeigt:
Eine perspektivische Darstellung des erfindungsgemässen-Implantats.

Das in der einzigen Figur dargestellte Zwischenwirbel-Implantat besteht im wesentlichen aus einem Hohlzylinder mit Innenraum 8, Längsachse 3, Deckfläche 1 und Grundfläche 2. Das Zwischenwirbel-Implantat besteht im wesentlichen aus einem polykristallinen, keramischen Werkstoff. Der keramische Werkstoff weist eine Porosität von 5 Vol.-% auf, wobei die Poren mit Luft gefüllt sind. Die Porenweite ist kleiner als 100 µm, vorzugsweise kleiner als 50 µm.
Der Fremdphasenanteil des keramischen Materials beträgt 1,5 Gew.-%. Die Druckfestigkeit des keramischen Werkstoffs beträgt 500 MPa.

Die Deck- und Grundflächen 1,2 sind für den Knochenkontakt zu den Deckplatten zweier Wirbelkörper bestimmt und entsprechend ausgebildet. Die Wandstärke des Zwischenwirbel-Implantats beträgt 7 mm und die Dichte des keramischen Werkstoffs beträgt 3,2. Die Deckfläche 1 und die Grundfläche 2 sind nicht planar ausgebildet, sondern sind mit einer Anzahl quer (d.h. radial) zur Längsachse 3 verlaufender Rillen 4 und Erhöhungen 5 versehen.
Die Deckfläche 1 und die Grundfläche 2 sind zueinander keilförmig zulaufend angeordnet und sind leicht nach aussen konvex gewölbt.

Im Mantel 6 des Zwischenwirbel-Implantats ist anterior eine Perforation 7 vorgesehen, welche der Aufnahme eines Manipulations-Instrumentes dient. Der Mantel ist ferner mit einer dreidimensionalen Strukturierung 9 versehen, welche eine Tiefe von 0,75 mm aufweist.

Nachstehend wird nun die klinische Anwendung des erfindungsgemässen Zwischenwirbelimplantats im Detail beschrieben.

Das in der einzigen Figur gezeigte Implantat wird mit Knochenspänen (bone graft oder Knochenersatzmaterial), eventuell unter Komprimierung derselben, gefüllt, mit einem geeigneten in die Perforation 7 eingeführten Instrument gefasst und unter Zuhilfenahme eines Distraktionsinstrumentes in den ausgeräumten Zwischenwirbelraum eingeführt.

## Patentansprüche

1. Zwischenwirbel-Implantat prismatischer oder zylindrischer Gestalt mit der Längsachse (3), dessen Deckfläche (1) und Grundfläche (2) für den Knochenkontakt zu den Deckplatten zweier Wirbelkörper geeignet sind, wobei das Zwischenwirbel-Implantat im wesentlichen aus einem porösen, keramischen Werkstoff besteht, der eine Porosität von höchstens 30 Vol.-% aufweist und dessen Poren mit Luft gefüllt sind und der Mantel (6) des Zwischenwirbel-Implantats mit einer dreidimensionalen Strukturierung (9) versehen ist,
**dadurch gekennzeichnet, dass** die
dreidimensionale Strukturierung (9) eine Tiefe von 0,5 - 1,0 mm aufweist.

2. Implantat nach Anspruch 1, **dadurch gekennzeichnet, dass** der keramische Werkstoff röntgentransparent ist.

3. Implantat nach Anspruch 1 oder 2, **dadurch gekennzeichnet, dass** die Deckfläche (1) und/oder die Grundfläche (2) nicht planar ausgebildet ist.

4. Implantat nach einem der Ansprüche 1 bis 3, **dadurch gekennzeichnet, dass** die Deckfläche (1) und/oder die Grundfläche (2) mit quer zur Längsachse (3) verlaufenden Rillen (4) und/oder Erhöhungen (5) versehen ist.

5. Implantat nach einem der Ansprüche 1 bis 4, **dadurch gekennzeichnet, dass** der Mantel (6) des Zwischenwirbel-Implantats mit einer oder mehreren Perforationen (7) versehen ist.

6. Implantat nach einem der Ansprüche 1 bis 5, **dadurch gekennzeichnet, dass** die Porosität des keramischen Werkstoffs höchstens 9 Vol.-% beträgt.

7. Implantat nach Anspruch 6, **dadurch gekennzeichnet, dass** die Porosität des keramischen Werkstoffs höchstens 5 Vol.-% beträgt.

8. Implantat nach einem der Ansprüche 1 bis 7, **dadurch gekennzeichnet, dass** die Wandstärke des Zwischenwirbel-Implantats mindestens 4 mm beträgt.

9. Implantat nach Anspruch 8, **dadurch gekennzeichnet, dass** die Wandstärke des Zwischenwirbel-Implantats mindestens 6 mm beträgt.

10. Implantat nach einem der Ansprüche 1 bis 9, **dadurch gekennzeichnet, dass** die Dichte des keramischen Werkstoffs grösser als 2,8 g/cm³ ist.

11. Implantat nach Anspruch 10, **dadurch gekennzeichnet, dass** die Dichte des keramischen Werkstoffs grösser als 3,1 g/cm³ ist.

12. Implantat nach einem der Ansprüche 1 bis 11, **dadurch gekennzeichnet, dass** das Zwischenwirbel-Implantat als hohler Kreiszylinder mit der Längsachse (3) ausgebildet ist.

13. Implantat nach einem der Ansprüche 1 bis 12, **dadurch gekennzeichnet, dass** die Deckfläche (1) und die Grundfläche (2) parallel zueinander angeordnet sind.

14. Implantat nach einem der Ansprüche 1 bis 12, **dadurch gekennzeichnet, dass** die Deckfläche (1) und die Grundfläche (2) keilförmig zueinander zulaufend angeordnet sind.

15. Implantat nach einem der Ansprüche 1 bis 14, **dadurch gekennzeichnet, dass** die Deckfläche (1) und/oder die Grundfläche (2) nach aussen konvex gewölbt ist.

16. Implantat nach einem der Ansprüche 1 bis 15, **dadurch gekennzeichnet, dass** das keramische Material polykristallin ist.

17. Implantat nach einem der Ansprüche 1 bis 16, **dadurch gekennzeichnet, dass** das keramische Material einen Fremdphasenanteil von kleiner als 3 vorzugsweise kleiner als 2 Gew.-% aufweist.

18. Implantat nach einem der Ansprüche 1 bis 17, **dadurch gekennzeichnet, dass** die Druckfestigkeit des keramischen Werkstoffs 400 - 600 MPa beträgt.

19. Implantat nach Anspruch 18, **dadurch gekennzeichnet, dass** die Druckfestigkeit des keramischen Werkstoffs 450 - 550 MPa beträgt.

20. Implantat nach einem der Ansprüche 1 bis 19, **dadurch gekennzeichnet, dass** die Porenweite kleiner als 100 µm ist.

21. Implantat nach Anspruch 20, **dadurch gekennzeichnet, dass** die Porenweite kleiner als 50 µm ist.

## Claims

1. Intervertebral implant, configured in prismatic or cylindrical form with a longitudinal axis (3), its top surface (1) and bottom surface (2) designed to serve as contact surfaces between the bone and the end plates of two vertebrae, the said intervertebral implant consisting essentially of a ceramic material, which has a porosity of 30 vol. % at the most, and whose pores are filled with air and that the jacket (6) of the intervertebral implant is provided with a three-dimensionally textured surface structure (9),
**characterized in that** the
three-dimensionally textured surface structure (9) has a depth of 0,5 - 1,0 mm.

2. Implant as in claim 1, **characterized in that** the ceramic material is transparent to X-rays.

3. Implant as in claim 1 or 2, **characterized in that** the top surface (1) and/or the bottom surface (2) is/are not planarly shaped.

4. Implant as in one of the claims 1 to 3, chracterized in that the top surface (1) and/or the bottom surface (2) is/are provided with grooves (4) and/or ridges (5) extending transversely to the longitudinal axis (3).

5. Implant as in one of the claims 1 to 4, **characterized in that** the jacket (6) of the intervertebral implant is provided with one or several perforation(s) (7).

6. Implant as in one of the claims 1 to 5, **characterized in that** the porosity of the ceramic material is limited to a maximum of 9 % by volume.

7. Implant as in claim 6, **characterized in that** the porosity of the ceramic material is limited to a maximum of 5 % by volume.

8. Implant as in one of the claims 1 to 7, **characterized in that** the wall thickness of the intervertebral implant is at least 4 mm.

9. Implant as in claim 8, **characterized in that** the wall thickness of the intervertebral implant is at least 6 mm.

10. Implant as in one of the claims 1 to 9, **characterized in that** the density of the ceramic material is greater than 2,8 g/cm³.

11. Implant as in claim 10, **characterized in that** the density of the ceramic material is greater than 3,1 g/cm³.

12. Implant as in one of the claims 1 to 11, **characterized in that** the intervertebral implant is configured as hollow circular cylinder extending along the longitudinal axis (3).

13. Implant as in one of the claims 1 to 12, **characterized in that** the top surface (1) extends parallel to the bottom surface (2).

14. Implant as in one of the claims 1 to 12, **characterized in that** the top surface (1) and the bottom surface (2) extend in wedge-shaped converging fashion in relation to each other.

15. Implant as in one of the claims 1 to 14, **characterized in that** the top surface (1) and/or the bottom surface (2) is/are curved in convex fashion toward the outside.

16. Implant as in one of the claims 1 to 15, **characterized in that** the ceramic material is polycrystalline.

17. Implant as in one of the claims 1 to 16, **characterized in that** the ceramic material has a foreign-phase content of less than 3 and preferably less than 2 % by weight.

18. Implant as in one of the claims 1 to 17, **characterized in that** the pressure resistance of the ceramic material is between 400 and 600 MPa.

19. Implant as in claim 18, **characterized in that** the pressure resistance of the ceramic material is between 450 and 550 MPa.

20. Implant as in one of the claims 1 to 19, **characterized in that** width of the pores is less than 100 µm.

21. Implant as in claim 20, **characterized in that** the width of the pores is less than 50 µm.

## Revendications

1. Implant intervertébral de configuration prismatique ou cylindrique, avec un axe longitudinal (3) et dont la surface de couvercle (1) et la surface de base (2) conviennent pour assurer le contact avec l'os des plaques de couvercle de deux corps vertébraux, l'implant intervertébral étant essentiellement constitué de matériau céramique poreux qui présente une porosité d'au plus 30 % en volume et dont les pores sont remplis d'air, l'enveloppe (6) de l'implant intervertébral étant dotée d'une structuration tridimensionnelle (9), **caractérisé en ce que** la structuration tridimensionnelle (9) présente une profondeur de 0,5 à 1,0 mm.

2. Implant selon la revendication 1, **caractérisé en ce que** le matériau céramique est transparent vis-à-vis des rayons X.

3. Implant selon la revendication 1 ou 2, **caractérisé en ce que** la surface du couvercle (1) et/ou la surface de base (2) ne présentent pas une configuration plane.

4. Implant selon l'une des revendications 1 à 3, **caractérisé en ce que** la surface du couvercle (1) et/ou la surface de base (2) sont dotées de rainures (4) et/ou de saillies (5) qui s'étendent transversalement par rapport à l'axe longitudinal (3).

5. Implant selon l'une des revendications 1 à 4, **caractérisé en ce que** l'enveloppe (6) de l'implant intervertébral est dotée d'une ou plusieurs perforations (7).

6. Implant selon l'une des revendications 1 à 5, **caractérisé en ce que** la porosité du matériau céramique vaut au plus 9 % en volume.

7. Implant selon la revendication 6, **caractérisé en ce que** la porosité du matériau céramique vaut au plus 5 % en volume.

8. Implant selon l'une des revendications 1 à 7, **caractérisé en ce que** l'épaisseur de la paroi de l'implant intervertébral vaut au moins 4 mm.

9. Implant selon la revendication 8, **caractérisé en ce que** l'épaisseur de la paroi de l'implant intervertébral vaut au moins 6 mm.

10. Implant selon l'une des revendications 1 à 9, **caractérisé en ce que** la masse spécifique du matériau céramique est supérieure à 2,8 g/cm³.

11. Implant selon la revendication 10, **caractérisé en ce que** la masse spécifique du matériau céramique est supérieure à 3,1 g/cm³.

12. Implant selon l'une des revendications 1 à 11, **caractérisé en ce que** l'implant intervertébral est configuré comme cylindre circulaire creux avec un axe longitudinal (3).

13. Implant selon l'une des revendications 1 à 12, **caractérisé en ce que** la surface de couvercle (1) et la surface de base (2) sont disposées parallèlement l'une à l'autre.

14. Implant selon l'une des revendications 1 à 12, **caractérisé en ce que** la surface de couvercle (1) et la surface de base (2) sont disposées de manière à s'étendre obliquement l'une par rapport à l'autre.

15. Implant selon l'une des revendications 1 à 14, **caractérisé en ce que** la surface de couvercle (1) et/ou la surface de base (2) sont bombées de manière convexe vers l'extérieur.

16. Implant selon l'une des revendications 1 à 15, **caractérisé en ce que** le matériau céramique est polycristallin.

17. Implant selon l'une des revendications 1 à 16, **caractérisé en ce que** le matériau céramique présente une teneur en phases étrangères inférieure à 3, de préférence inférieure à 2 % en poids.

18. Implant selon l'une des revendications 1 à 17, **caractérisé en ce que** la résistance en pression du matériau céramique vaut de 400 à 600 MPa.

19. Implant selon la revendication 18, **caractérisé en ce que** la résistance en pression du matériau céramique vaut de 450 à 550 MPa.

20. Implant selon l'une des revendications 1 à 19, **caractérisé en ce que** les pores ont une taille inférieure à 100 µm.

21. Implant selon la revendication 20, **caractérisé en ce que** les pores ont une taille inférieure à 50 µm.
